# EUROPEAN PATENT APPLICATION

(11) **EP 0 611 582 A2**
(43) Date of publication of application: **24.08.1994**
(21) Application number: 94100730.4
(22) Date of filing: 19.01.1994
(51) Int. Cl.: A61M 29/02, A61M 1/10

(54) **Single-lumen over-the-wire IAB catheter**

(30) Priority: 19.01.1993 US 5563
(71) Applicant: DATASCOPE INVESTMENT CORP., Montvale, N.J. 07645 (US)
(72) Inventor: Wilcox, Robert L., Wanaque, New Jersey 07465 (US); Schock, Robert B., Sparta, New Jersey 07871 (US); Lucas, John J., Sparta, New Jersey 07871 (US); Kaiser, Walter, Ramsey, New Jersey 07446 (US)
(74) Representative: Schmidt-Evers, Jürgen, Dipl.-Ing.

(57) **Abstract**

Intra-aortic balloon catheters for placement using an indwelling guide wire having guide structures which reduce the size of the catheter, which can increase distal perfusion and facilitate insertion and placement. These guide structures include short internal or external lumens, formed from separate guide sleeves (15, 25, 73) or by the method of wrapping the balloon membrane (7) itself. The guide sleeves (15, 25, 73) can be located on or inside the balloon membrane (7) or can be attached to the balloon tip or catheter tube (3), and can be permanent or detachable.

## Description

### BACKGROUND OF THE INVENTION

### Field of the Invention

This invention relates to intra-aortic balloon ("IAB") catheters used to perform intra-aortic balloon pumping ("IABP"). More particularly, it pertains to novel intra-aortic balloon catheters which have a single lumen, yet which can still be used with an indwelling guide wire.

### Description of the Related Art

Life-threatening cardiac problems may reduce the ability of a patient's heart to pump blood. When the heart continues pumping, albeit in a weakened manner, the weakened heart may be benefitted by lessening the load thereon.

One popular support system which can assist a weakened heart is intra-aortic balloon pumping ("IABP"). IABP is performed using a non-distensible balloon (the "IAB") which has been positioned in the patient's aorta. By filling the balloon with fluid it can be controllably expanded, and by removing the fluid it can be deflated. Fluid exchange is accomplished through a catheter running from the balloon out of the patient's body.

In many instances, particularly whenever the IAB is to be used in a patient suffering from sclerotic disease, or a patient in which the blood vessels through which the IAB will pass are highly tortuous, a thin, flexible guide wire may first be used to negotiate a path to the site where the IAB is to be placed, which may be the descending aorta. Once the guide wire is properly positioned, it serves as a path leading the IAB to the proper site. Of course, the catheter must be designed to cooperate with the guide wire.

Known IAB catheters suitable for use with a guide wire are constructed with a second lumen through which the flexible guide wire passes, and are commonly referred to as "dual-lumen catheters". Examples of dual-lumen catheters are disclosed in U.S. Patent No. 4,261,339, to Hanson et al. Typically, this second lumen communicates with an opening at the distal tip of the IAB balloon. The second lumen and guide wire are dimensioned to allow relative lengthwise motion therebetween. To position this type of IAB, the guide wire is inserted into the patient and advanced into the patient's aorta, using known medical procedures which need not be described. The guide wire is long enough so that a length of wire extends outward from the patient. Either before or during the medical procedure, the proximal end of the guide wire is inserted into the guide wire lumen through the opening at the distal tip of the balloon. Now, the IAB is advanced along the guide wire and into the patient through either an insertion sheath located in a puncture or through a pre-dilated puncture without a sheath into the patient. The IAB follows the guide wire through the arterial tree until it reaches the descending aorta. Typically, the puncture wound through which the physician gains access to the arterial system (normally the femoral artery) is made in the patient's groin.

The catheter tube is designed with at least one lumen which allows fluid to flow therethrough into the balloon. After the balloon inflates, it is deflated by evacuating the fluid back through the lumen. The balloon is non-distensible.

The balloon is periodically inflated in synchronization with the patient's own heartbeat, increasing the blood flow rate. Ideally, the balloon is inflated out of phase and in counterpulsation to the heartbeat. This significantly reduces the load on the heart.

As previously noted, axial force must be applied to the IAB to push it through the arterial tree into the aorta. Because the route to the aorta can be fairly tortuous, a substantial load may have to be applied. If the catheter is not stiff enough to support this load, buckling may occur. It is important to avoid buckling, because a buckled catheter may have to be replaced, a time-consuming procedure.

Since the IAB passes through the arterial system, it is desirable to minimize blood flow obstructions. In those instances where the IAB is to be inserted using an insertion sheath, to increase distal blood flow and reduce trauma, it is also desirable to minimize the size of that insertion sheath through which the IAB passes into the patient. Since even the deflated IAB is fairly bulky, much work has gone into reducing the size of the deflated balloon so that its deflated cross-sectional area is approximately the same as that of the catheter. This reduces the necessary size of the arterial puncture, and the insertion sheath, if an insertion sheath is to be used.

In part, deflated IAB balloon size is reduced by twisting or wrapping the deflated balloon relative to the catheter tube. Different wrapping techniques are shown in U.S. Patent No. 4,261,339 to Hanson and Wolvek, and U.S. Patent No. 4,576,142 to Schiff.

Once the cross-sectional area of the deflated balloon is reduced to approximately that of the catheter, any further reduction in deflated balloon area is pointless, since the larger catheter immediately following the balloon would present the same problems as would the larger balloon.

Catheter diameter can also be reduced by eliminating the guide wire and the associated lumen. In particular, the guide wire lumen increases the size of the IAB catheter tube.

Because some patients such as women and Japanese have inherently narrow and/or tortuous blood vessels, or have blood vessels so narrowed by sclerotic disease that dual-lumen IAB's are not feasible, these cases require the use of single-lumen IAB's. As previously noted, single-lumen IAB's can have smaller profiles than dual-lumen IAB's. However, because these single-lumen devices do not ride over an indwelling guide wire, they may have difficulty navigating the extreme tortuosity found in patients with advanced sclerotic disease.

There is a limit to which catheter size can be reduced, because it is necessary to achieve a minimum fluid flow rate to insure that the IAB is inflated and deflated at a rate which is a function of the patient's heartbeat rate.

### SUMMARY OF THE INVENTION

Among the features of the present invention is an intra-aortic balloon catheter having an inflatable balloon membrane having a balloon chamber, a catheter tube having a catheter tube lumen, the tube lumen being in fluid communication with the balloon chamber, and a guide sleeve having a lumen through which a guide wire may pass. The guide sleeve passes through the balloon membrane toward the distal end of the balloon and both the proximal and distal ends of the balloon membrane are attached to the corresponding portions of the guide sleeve in a fluid-tight manner.

Another embodiment of the present invention involves an intra-aortic balloon catheter which contains an inflatable balloon membrane, a catheter tube having a catheter tube lumen, and a guide sleeve having a guide wire lumen. The guide sleeve is affixed to the outside of the balloon membrane without the guide sleeve passing through the balloon membrane.

Still a further embodiment of this invention relates to an intra-aortic balloon catheter which has an inflatable balloon membrane, a catheter tube having a catheter tube lumen, the tube lumen being in fluid communication with the balloon chamber, and a tubular guiding tip member having a guide wire lumen. The tip member has a proximal opening and a distal opening, each communicating with the guide wire lumen, and the proximal end of the tip member is attached to the distal end of the balloon membrane, so that fluid cannot leak out from the balloon chamber into the guide wire lumen.

Another embodiment of this invention is a retractable guiding tip for a catheter which includes a shield collar having a cylindrical inner region, a tubular guiding member that is slidably received within the inner region so that the tubular guiding member can reciprocally move into and out of the shield collar, and a stop attached to either the shield collar or to the tubular guiding member. The stop limits movement of the tubular guiding member into the shield collar. The retractable guide tip allows the size of the IAB to be reduced.

Additionally, this invention encompasses an intra-aortic balloon catheter that contains an inflatable balloon membrane, a catheter tube having a catheter tube lumen, the tube lumen being in fluid communication with the balloon chamber, and a retractable guiding tip. This guiding tip has a shield collar with a cylindrical inner region. Also provided is a tubular guiding member, the tubular guiding member being slidably received within the inner region so that the tubular guiding member can reciprocally move into and out of the shield collar. A stop is attached either to the shield collar or to the tubular guiding member, so as to limit movement of the tubular guiding member into the shield collar. The retractable guiding tip is disposed at the distal end of the balloon membrane, and the guiding member and shield collar are dimensioned so that regardless of how the guiding member is positioned, the balloon chamber is not in fluid communication with an environment outside the balloon chamber. This retractable tip helps reduce the size of the IAB.

Still a further embodiment of the present invention regards an intra-aortic balloon catheter that contains a balloon membrane, a catheter tube having a tube lumen, the proximal end of the balloon membrane being attached to the catheter tube in fluid-tight manner, and a guide sleeve having a guide wire lumen. The guide sleeve is releasably attached to the catheter tube so that the guide sleeve can be detached from the catheter tube and removed.

Also encompassed by this invention is an intra-aortic balloon catheter which includes a balloon membrane, a catheter tube having a tube lumen, the proximal end of the balloon membrane being attached to the catheter tube in fluid-tight manner, a first guide body mounted on balloon membrane distal end, and a second guide body mounted on one of the proximal end of the balloon or a portion of the catheter tube adjacent to the proximal end of the balloon membrane.

Further, the present invention extends to an intra-aortic balloon having a balloon membrane, a catheter tube having a tube lumen, the proximal end of the balloon membrane being attached to the catheter tube in fluid-tight manner, and a collapsible inner guide sleeve having a proximal opening, distal opening, and guide wire lumen for accommodating a guide wire running between the proximal and distal openings. The distal opening is disposed by the distal end of the balloon membrane, and the proximal opening of the inner tube is disposed within the catheter tube lumen. Sealing means prevents fluid from passing through the guide wire lumen when the guide wire is removed from the guide sleeve.

In addition, this invention relates to a single-lumen, over-the-wire intra-aortic balloon which includes a catheter tube having a tube lumen, and a balloon membrane. The proximal end of the balloon membrane is attached to the catheter tube in fluid-tight manner, and the balloon membrane is shaped to form an open passage approximately parallel to the balloon axis. A guide wire can be inserted through the open passage.

Yet another embodiment of this invention is a method of guiding an intra-aortic balloon catheter by providing an intra-aortic balloon catheter having a balloon membrane, and shaping the balloon membrane to form an open passage which will accommodate a guide wire. By causing the open passage to surround the guide wire the balloon catheter can be advanced while the open passage surrounds the guide wire.

### BRIEF DESCRIPTION OF THE DRAWINGS

The invention described in this application can be better appreciated with reference to the following Figures, in which like numbers identify like parts in the various views. These Figures are means to be exemplary and non-limiting; accordingly, they should not be understood to limit the scope of the claimed invention.
FIG. 1 is a side cross-sectional view of a monorail IAB in which the guide sleeve passes through the balloon;
FIGS. 2A and 2B are front cross-sectional views of the device shown in FIG. 1, taken along lines A-A' and B-B', respectively;
FIG. 3 is a side cross-sectional view of a monorail IAB in which the guide sleeve is attached to the outside of the balloon chamber;
FIG. 4 is a front cross-sectional view of the device shown in FIG. 3 taken along lines A-A';
FIG. 5 is a top cross-sectional view of an IAB balloon having a straight guiding tip;
FIG. 6 is a side cross-sectional view of the device shown in FIG. 5 taken along line A-A';
FIG. 7 is a front cross-sectional view of the device shown in FIG. 6 taken along line B-B';
FIG. 8 is a top view of an IAB having a pig-tail guiding tip.
FIG. 9 is a side cross-sectional view of the device depicted in FIG. 8 taken along line A-A';
FIG. 10 is a front cross-section view of the device shown in FIG. 9 taken along line B-B';
FIG. 11 is a side view of IAB having guide sleeves joined to balloon tip and proximal balloon attachment points;
FIGS. 11A and 11B are front cross-sectional views of the device depicted in FIG. 10 taken along lines A-A' and B-B', respectively;
FIG. 12 is a side cross-sectional view of an IAB balloon tip having an oblique guide wire lumen;
FIG. 13 is a side cross-sectional view of an IAB balloon tip having a curved guide wire lumen;
FIG. 14 is a side cross-sectional view of a retractable IAB guide wire tip in the extended position;
FIG. 15 is a side cross-sectional view of a retractable IAB guide wire tip in the retracted position;
FIG. 16 is a side cross-sectional view of an IAB having a collapsible guide sleeve;
FIG. 17 is a close-up side view of slit valve used with the collapsible guide sleeve;
FIGS. 18A and 18B are cross sectional views of two different balloon wrapping configurations for single-lumen over-the-wire IAB's;
FIG. 19 is a side cross-sectional view of a detachable lumen IAB; and
FIG. 20 is a front cross-sectional view of the detachable lumen IAB.

### DESCRIPTION OF THE PREFERRED EMBODIMENTS

Since all of the devices which will now be described are to be used in medical procedures, it is essential that such devices be made from materials which, when placed inside the body, are sufficiently inert to avoid provoking a thrombogenic response. It is also important that materials be used which can withstand the conditions found inside the human body. Lastly, the devices made according to this invention must be sterilizable using at least one, and preferably any, of the accepted sterilization procedures for medical devices.

Suitable materials include stainless-steel, and plastics, particularly polyurethane, polyester, Teflon® (polytetrafluouro-ethylene) and polyethylene.

The present invention has been developed in accordance with the inventors' observation that the improvement of IAB performance, kink-resistance and high pushability should be combined with the smallest possible catheter profile and most flexible catheter body.

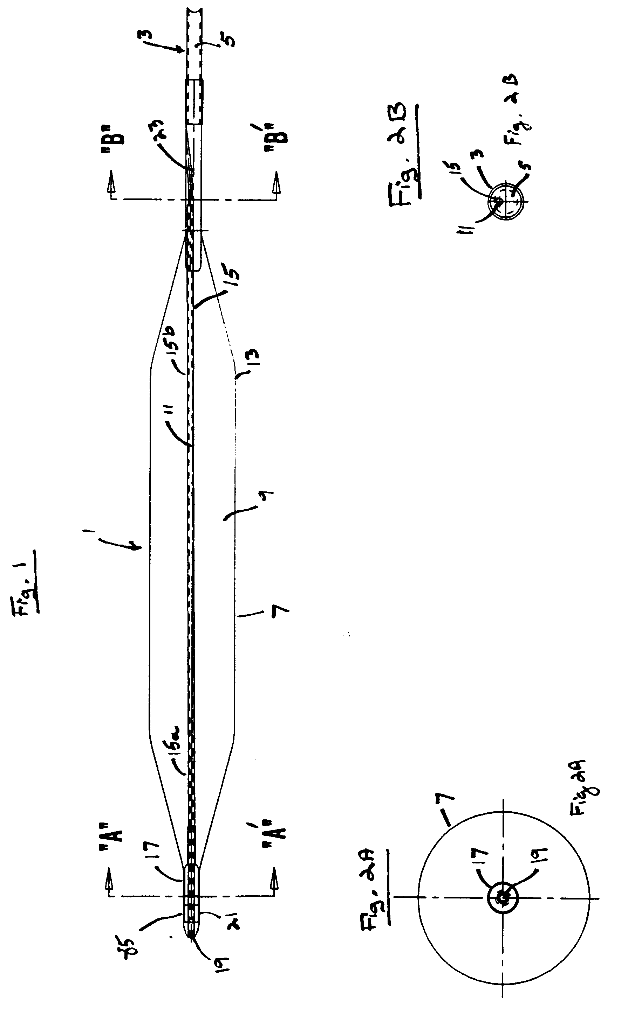

As depicted in Figs. 1-2b, one embodiment of the present invention involves an IAB 1 in which the catheter tube 3 has only a single lumen 5, while the balloon 7 itself has a chamber 9 and lumen 11. Typically, the inflated balloon is a 40 cc polyurethane balloon 13 of standard design. While polyurethane has been found to work well as a balloon membrane, those of ordinary skill in the art will appreciate that numerous other materials can be used instead.

One benefit of eliminating the guide wire lumen from the catheter tube 3 is that the diameter of the catheter tube can be reduced. As previously noted, this is highly desirable, since a smaller diameter catheter tube allows more room for blood flow to the distal vasculature. In addition, a smaller catheter tube can pass through a smaller introducer sheath, or if no sheath is used, through a smaller pre-dilated puncture (i.e., femoral artery puncture). Accordingly, the present invention is in part concerned with providing small-diameter single-lumen IAB catheters.

The balloon chamber 9 defined by the balloon membrane communicates with the catheter tube lumen 5. The balloon membrane 7 is affixed to the catheter tube 3 in fluid-tight fashion. A flexible inner guide sleeve 15 passes through the chamber 9 and the interior of this sleeve is provided with lumen 11, through which the guide wire can pass. The sleeve is used together with a guide wire that is about 150 cm long, and made of .018" or .030" diameter Teflon® - coated stainless steel. Of course, other size wires can be used. To improve balloon navigability, and to protect against damage to the artery, the guide wire (not shown) can have an elongated flexible tip that is shaped like a "J" or pig tail (helical spiral). Those of ordinary skill in the art will be aware of the various guide wires constructions which are available. The catheter tip 17 has a lumen 19 which allows it to ride over the guide wire. Because it is imperative that the fluid integrity of this second lumen not be compromised by rip, tear or wear, this guide sleeve 15 is preferably made with a front section 15a which is about 8" long, .023" ID, .042" OD polyurethane material. This is attached to a second section 15b which is about 2" long, .020" ID, .045" 00 polyurethane material. The second section 15b of guide sleeve 15 passes into the catheter tube lumen 5 and leads to a side channel 23. Channel 23 is open at its proximal end to the external environment. The first section 15a of guide sleeve 15 is connected to the distal tip 17 of the balloon. The tip 17 of the balloon 13 has a lumen 19 which communicates with lumen 11 and through which the guide wire can pass, is a standard 9.5 Fr. tip, made of polyurethane, with a radio-opaque marker 21; of course, other dimensions and materials could be used for these parts, and a single piece guide sleeve could be used.

The catheter attached to the IAB is preferably about 21" long, .072" ID, .101 OD" polyurethane.

The point where the catheter tube 3 meets the balloon membrane 7 is carefully prepared to insure a strong, fluid-tight bond between the two materials. Similarly, the flexible tip 17 and balloon membrane 7 are attached in fluid-tight fashion. Both of these joints can be prepared using techniques well-known to those of ordinary skill in the art, such as heat welding, radio-frequency welding, solvent-welding, pressure welding, ultrasonic bonding, or adhesives.

To inflate the IAB 1, working fluid, typically carbon dioxide or helium gas, is shuttled into and out of the balloon chamber 9 via the catheter tube lumen 5. To avoid fluid leakage into the patient's body, the connections between the balloon membrane 7 and the catheter tube 3, and between the balloon membrane and tip 17, must be fluid-tight.

The IAB distal tip 17 can be constructed to help guide the IAB 1 during insertion. The tip 17 can be formed either by making the inner guide sleeve 15 long enough to protrude beyond the balloon membrane 7, and smoothing the tip of the inner guide sleeve to facilitate insertion, or by providing a separate tip member to which the balloon membrane and inner guide sleeve are both attached in fluid-tight manner. Of course, both of these tip constructions should have a lumen 19 that is suitably dimensioned and positioned to allow passage of the guide wire.

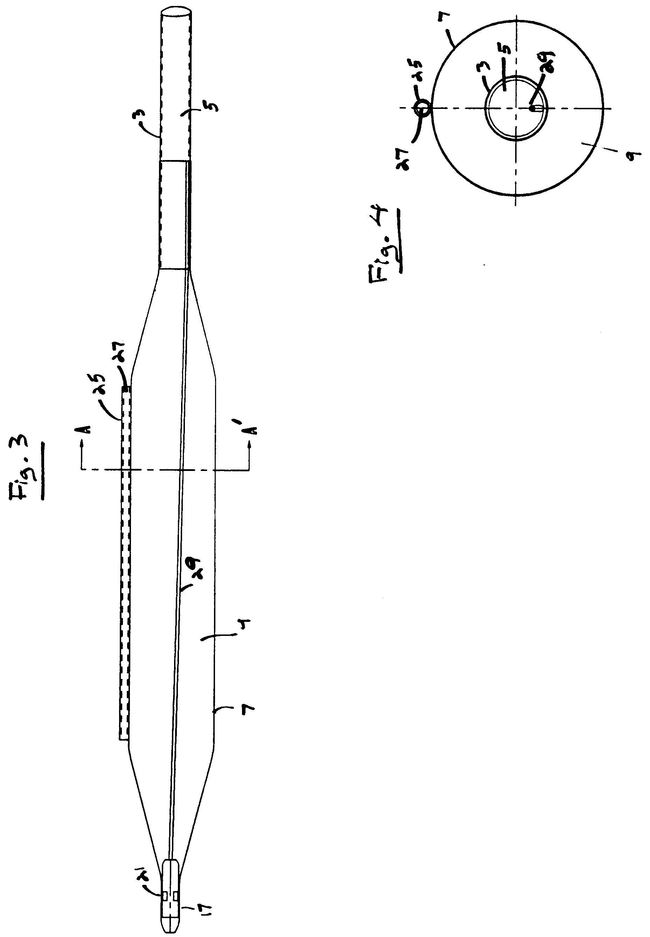

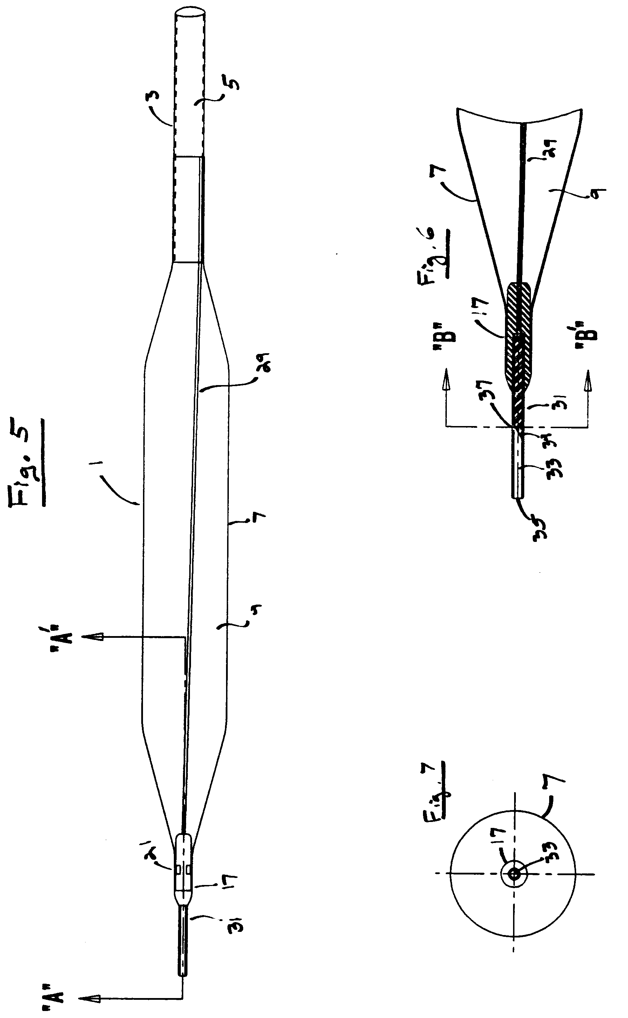

As depicted in Figs. 3 and 4, an alternate embodiment of this invention employs an external guide sleeve 25 attached to the exterior of the balloon membrane 7 or at some other point on the catheter to form an external guide structure. The guide sleeve 25 is preferably an integral part of the balloon membrane, but can also be formed as a separate structure which is later attached to the device. The inner lumen 27 of the guide sleeve is dimensioned to allow passage of a guide wire. The guide sleeve should be dimensioned to accommodate the largest guide wire which may be used, and also allow removal of that guide wire therethrough. Of course, the lumen can be shorter or longer than the balloon, and other methods of attachment such as ultrasonic welding can be employed.

A stiffening wire 29 is provided to transfer the axial force exerted by the physician on the catheter tube 3 to advance the IAB, from the catheter tube 3 to the tip 17.

Figs. 5-7 illustrate an embodiment of this invention which is a variation on the aforementioned external guide sleeve embodiment. In this embodiment, rather than provide a guide sleeve running along the side of the balloon membrane, the guiding structure 31 is provided at the balloon's distal tip.

Specifically, a guiding structure 31 is provided at the distal end of the IAB balloon 1. This guiding structure 31 is approximately .8"-1.0" in length (other lengths can, of course, be used) and it is attached to the tip 17. Any suitable technique such as heat welding can be used to join the guiding structure 31 and tip member 17. It is also possible to provide a unitary element which combines the guiding structure and tip member, and which unitary structure is attached to the balloon 7 so that the guiding tip member forms an integral part of the balloon.

Regardless of how it is formed, the guiding structure 31 has a guiding lumen 33 running from a front opening 35 to a rear opening 37 disposed toward the proximal end of the guiding structure near where it meets (or becomes) the tip 17. It will be seen in Fig. 6 that the inside of the guiding structure 39 has an inclined surface 39 which helps to lead a guide wire from the front opening 35 to the rear opening 37; if desired, this inclined surface can be omitted. Since it may be desirable to withdraw the guide wire from the IAB once the IAB has been properly positioned, particularly when the IAB is left in the patient for a substantial period of time, it will be appreciated that the front and rear openings 35, 37 and guiding lumen 33 must be sufficiently large so that when the guide wire is withdrawn, the guide wire, including any enlarged guiding tip portion, can pass therethrough without restriction. The IAB structure is otherwise similar to that previously described, and includes a stiffening wire 29.

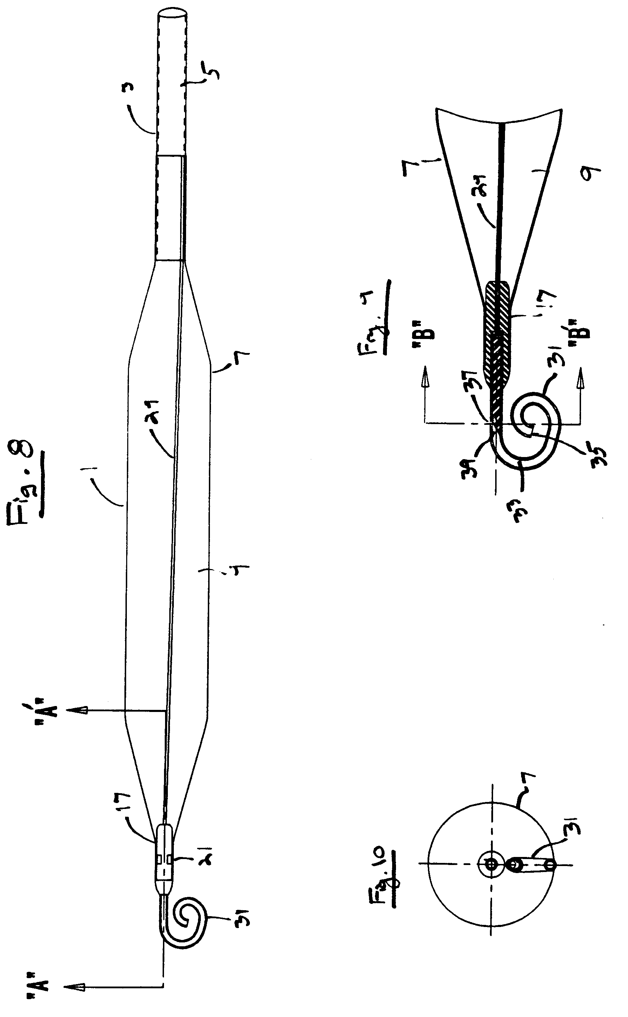

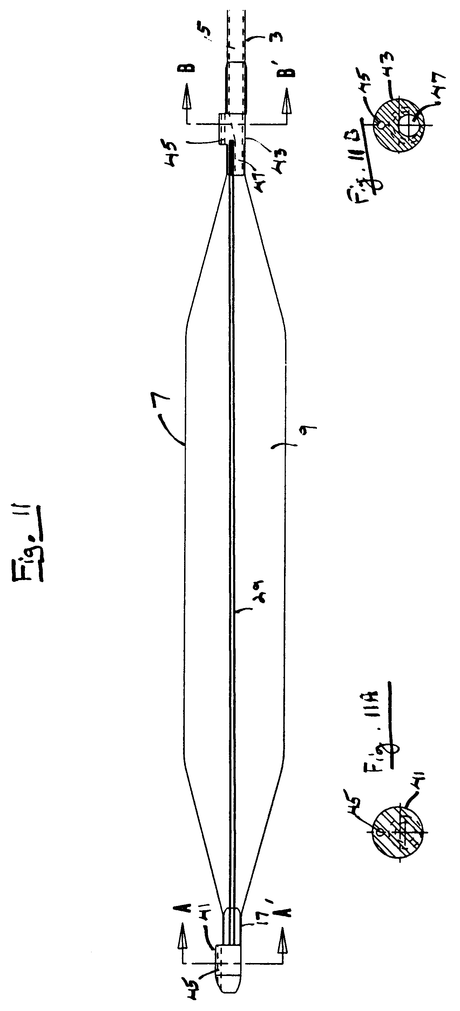

In a further refinement of this guiding structure, as shown in Figs. 8- 10, the IAB 1 is provided with guiding structure 31 which is a tube made from flexible material having shape memory. Preferably, such a tube can be from about .5 cm to 8 cm long, with the particular length which is to be used being dictated by the conditions encountered. It should be understood that these dimensions are provided as examples of the best way of practicing this invention, and that the invention is not to be limited to devices of these sizes.

During fabrication of the device, the guiding structure 31 is caused to take on and "remember" a helically curving shape so that if not constrained it spirals in on itself. The extent to which the guide structure spirals can be varied, but it may be preferable for it to wrap around in a helix for several turns, like a pig's tail. This is desirable because the helical spiral allows a fairly long guide structure to be contained within a fairly small space.

Either prior to (i.e., in a pre-loaded IAB) or during use, the guide structure is slid over the proximal end of an indwelling guide wire (this guide wire is already positioned in the patient) and then into the patient. The wire passes through a front opening 35 along a lumen 33, and out via a rear opening 37. Again, an inclined surface 39 can be provided to help guide the wire out through the rear opening 37; if desirable, this structure can be omitted. Again, front and rear openings 35 and 37 and lumen 33 may be suitably dimensioned to allow withdrawal of the guide wire and guide wire tip structure therethrough. The additional support provided by the guide wire causes the "pig tail" to straighten out and follow the guide wire through the arterial tree.

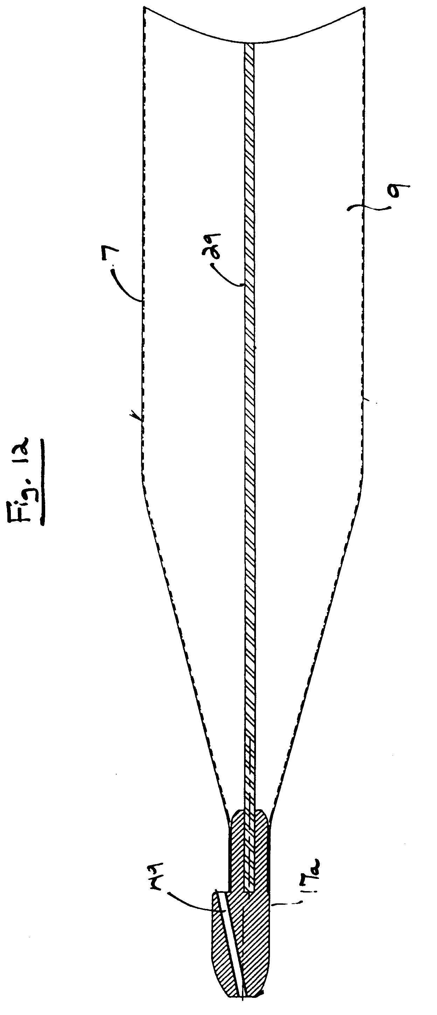

An alternate embodiment of this invention provides a guide body or bodies 41, 43 each having a guide wire lumen 45, as shown in Figs. 11, 11A and 11B.

As shown in Figs. 11-11B, the balloon membrane 7 is attached to tip 17, and tip 17 is attached to front guide body 41. However, it is also possible to form the tip and guide body as an integral piece. Similarly, the balloon membrane 7 and catheter tube 3 are each attached to rear guide body 43. As shown in Fig. 11, rear guide body 43 includes a fluid lumen 47 that allows the catheter tube lumen 3 to communicate with balloon chamber 9. If the guide wire is to be withdrawn from the patient, then it will be necessary to dimension the guide wire lumens 45 to allow the guide wire to pass therethrough.

In addition, a stiffening wire 29 is used to transmit axial force to tip 17. This stiffening wire 29 connects the front and rear guide bodies 41 and 43, and can be attached to those bodies in any suitable manner.

In this regard, it will be appreciated that tip 17 can be shaped in many ways; the main concern is that the shape not complicate balloon placement. For example, the tip could be curved or bent so that the guide wire lumen 45 is displaced from the balloon central axis, and so that the distal end of tip 17 then curves back to approximately the balloon central axis. Alternatively, the distal end of tip 17 might not curve back.

If the guide body is provided at just one point on the IAB, it may be preferable to locate this body at the distal tip of the IAB, since this better guides the IAB throughout the insertion process. IAB's having just a distal guide sleeve have already been discussed.

The manner in which these guide wire bodies are formed is not critical; the embodiments just discussed could be made from machined or molded plastic. They also can be made by providing separate tubes of suitable composition which are bonded to the catheter balloon or the catheter tube. Alternatively, the guide wire bodies can be formed integrally in the balloon tip. Likewise, a guide wire body could be provided at the proximal balloon attachment point by attaching a collar to the outside of the catheter. This collar is affixed to the catheter tube at or just proximal to the point where the balloon meets and is bonded to the catheter tube so that it cannot move.

It will be appreciated that in each of the above-described embodiments of the invention, the guide sleeve can be lengthened or shortened without departing from the scope of the invention. For example, in the embodiment corresponding to Fig. 3, the guide sleeve could be shortened, or could be attached at intermediate positions along the balloon.

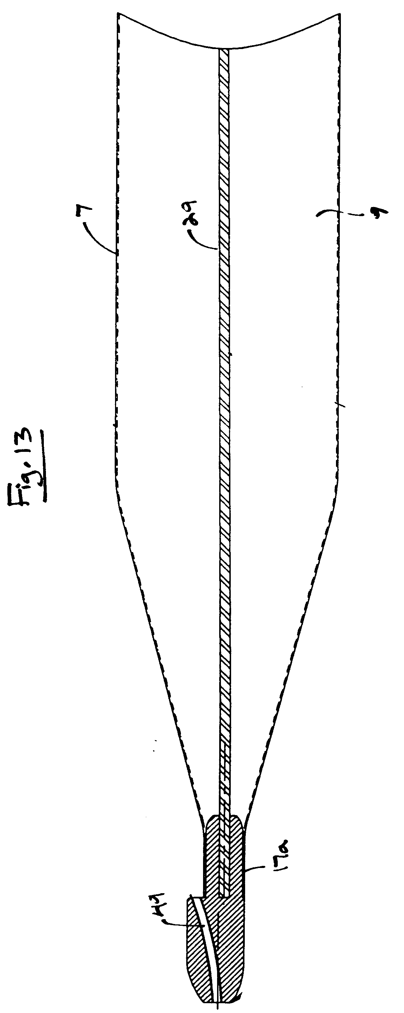

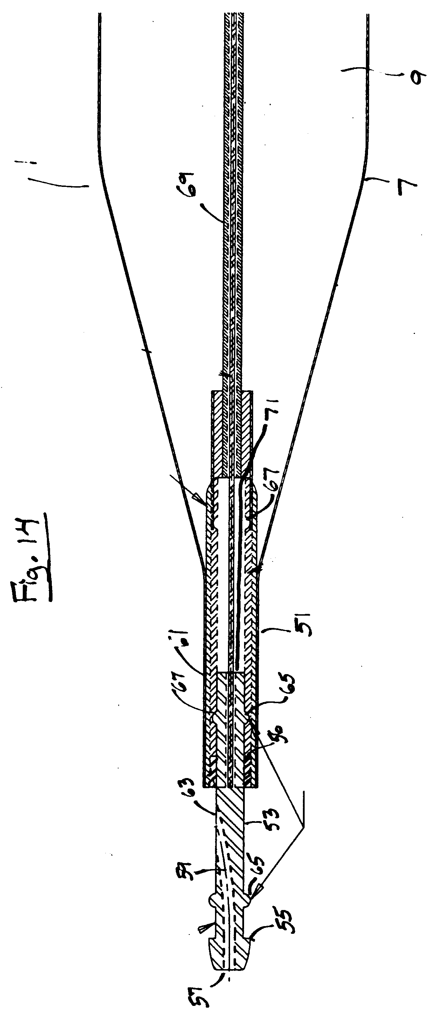

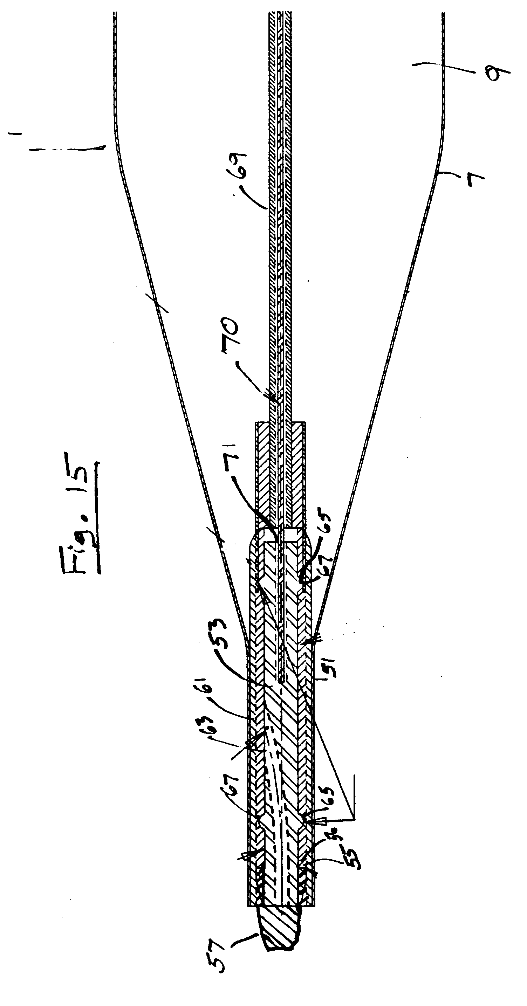

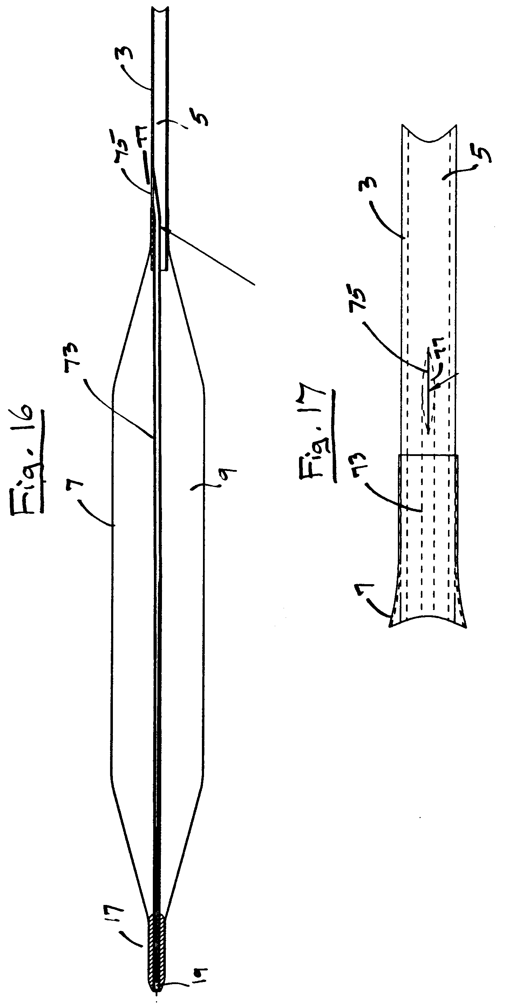

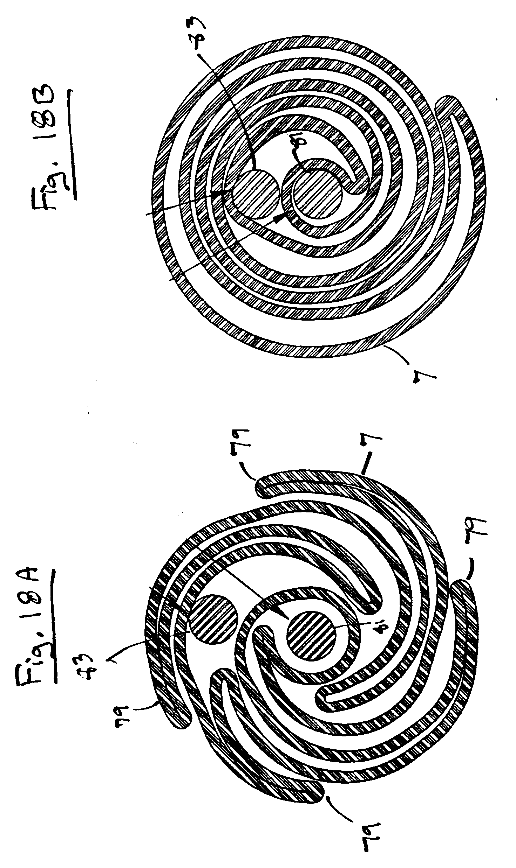

An alternate tip structure 17a is depicted in Figs. 12 and 13. The tip, which is affixed to the balloon membrane in fluid-tight manner, has a guide wire lumen 49 which allows a guide wire to pass therethrough. This lumen 49 can be a straight line which is not parallel to the balloon axis as shown in Fig. 12, or be curved as in Fig. 13. It could also be parallel to the balloon axis. The lumen is oriented in this manner so that a guide wire inserted therethrough can pass out of the proximal side of the tip structure while clearing the balloon without kinking.

It will be appreciated that in any of the foregoing embodiments, the stiffening wire could be extended to continue through the catheter, although such a wire may increase the catheter diameter by about 1/2 Fr.

Once an IAB having a guiding tip structure is positioned in the aorta, the guiding tip serves no purpose, Therefore, the inventors have provided a refinement of this invention having a retractable guiding tip structure 51. During placement, this tip helps reduce the likelihood that the IAB will enter the left subclavian artery; during withdrawal, the tip is retracted, reducing the size of the IAB.

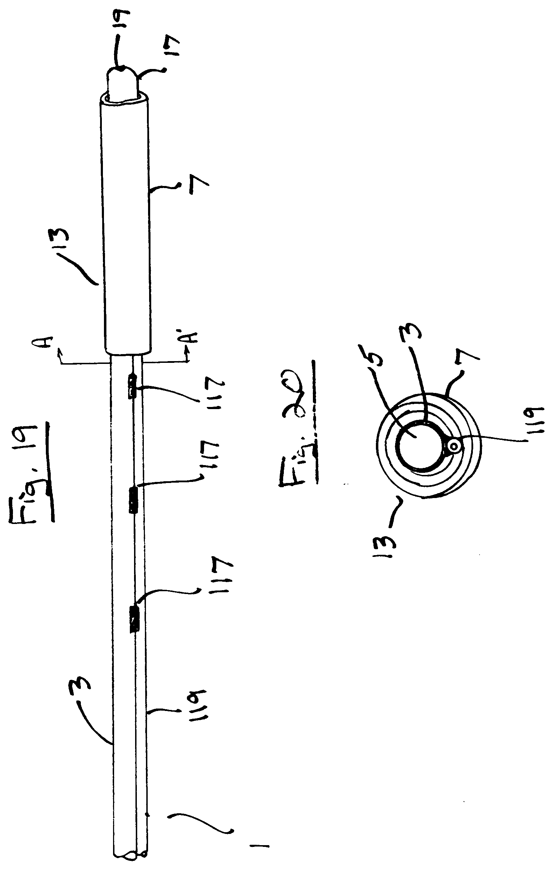

This retractable guide tip 51 is shown in Figs. 14 and 15. The retractable guide tip 51 is depicted with the movable tip 53 extended, as it would be just before the device is mounted on a guide wire. The retractable tip assembly 51 is mounted in a single-lumen IAB catheter 1. The movable tip 53 is tubular, with a flange 55 at its tip. The movable tip 53 has an open distal end 57 allowing communication with the guide wire lumen 59. The movable tip 53 is held in a slide collar 61, which contains the tip in a way that allows axial movement of the tip in the proximal direction. The balloon membrane 7 is attached to the slide collar 61 using any suitable attachment technique, such as adhesive bonding or welding.

A side port 63 is provided in the side of the movable tip 53 so that a guide wire can pass through the opening in the distal end 57 of the retractable tip, continue through the guide wire lumen 59, and then emerge from the side port 63. The movable tip 53 is long enough to insure that even when fully extended to allow a guide wire to pass therethrough, enough of the tip 53 remains in the slide collar 61 to securely hold the tip without undue movement, and to prevent fluid leakage into or out of the balloon chamber 9.

To prevent fluid in the balloon chamber 9 from leaking through the retractable tip structure into the blood or pull wire sheath 69, seal rings 65 can be provided which seat in grooves 67 when the tip is extended or retracted. Alternatively, the tip 53 can be dimensioned so that when it is fully retracted in the slide collar 61, the back of the tip 53 seats against structure in the slide collar, preventing leakage therebetween.

Likewise, the flange 55 at the distal end of the tip 53 prevents the tube from being pulled too far into the slide collar 61.

A thin pull wire 70, 0.010" in diameter, runs through a sheath 69 into the proximal end of the IAB catheter 1 (not shown) along the balloon chamber, and is affixed to the proximal end of the tip 71 in any appropriate manner. A suitable length, perhaps 1 foot, of this wire 70 protrudes from the proximal end of the catheter tube, and is affixed to a pull handle (not shown).

This device is inserted into the patient in the same manner as the embodiment of the invention having a flexible guiding tip structure previously described. IABP can be performed in the same manner as with other IAB devices.

After the IAB has been introduced over a guide wire, the guide wire can be withdrawn from the patient. The guide wire lumen 59 is dimensioned so that during guide wire removal, all parts of the guide wire, including any enlarged tip guiding structure, can pass therethrough. Next, the movable tip 53 is drawn into the IAB balloon chamber 9 when the physician pulls on a handle (not shown) attached to the pull wire 70. Of course, the force required to retract the tip 53 must not be so great that the catheter tube or balloon buckles or that the pull wire 70 breaks.

The tip 53 is constructed in two ways that facilitate retraction. As previously noted, the distal end of the tip has a flange 55 that engages a shoulder 56 at the distal end of the slide collar 61. As shown, a rounded portion of the tip 53 protrudes from the slide collar 61; of course, the slide collar could be dimensioned to extend over more or even all of this tip. In addition, once the flange 55 and shoulder 56 engage, tension exerted along the pull wire 70 is transferred to the distal end of the balloon, pulling the balloon backward. The flange 55 prevents the tip guide 53 from being pulled too far into the balloon chamber 9.

Second, small sealing rings 65 protrude from the exterior of the tubular member which forms the movable tip 53. When the tip 53 is pulled into the slide collar 61 by the force exerted by the pull wire 70, these sealing rings 65 slide and engage suitably dimensioned grooves 67 inside the interior of the slide collar 61. This prevents gas from entering the blood.

Although these sealing rings 65 can be omitted, they may make the device more secure.

It is also possible to design the retractable tip guide structure so that when the tip 53 is pulled into the slide collar 61, the proximal end of the tip contacts and seals against the sheath 69 and/or a shoulder surface, preventing fluid in the balloon chamber 9 from flowing into the sheath 69 or out of the balloon.

Alternatively, an IAB balloon can be designed with a collapsible guide sleeve. In the absence of a guide wire, this collapsible guide sleeve will collapse and so obstruct gas flow through tube lumen 5 less than a conventional sleeve.

For example, as shown in Figs. 16 and 17, the collapsible guide sleeve 73 runs between the guide tip 17 and the proximal portion of the balloon 7. The guide tip 17 is attached to both the balloon membrane 13 and the collapsible guide sleeve 73 in fluid-tight manner. The guide tip 17 is mounted so that its lumen 19 can communicate with that of the collapsible guide sleeve.

The proximal end of the balloon membrane 7 is attached in fluid-tight manner to the catheter tube 3. This allows fluid to pass from the catheter tube lumen 5 into the balloon chamber 9. The proximal end of the collapsible guide sleeve 73 exits through the side wall of the catheter tube lumen 5 and is attached to the inner wall of the catheter tube 3. The proximal end of the guide sleeve is open and communicates with the environment outside catheter tube 3 through an opening 77 in the catheter tube wall 5. A slit valve 75 is provided in this opening 77. The slit valve 75 can be an integral part of either the collapsible guide sleeve 73 or the catheter tube 5, or can be a separately-formed structure. Thus, a guide wire (not shown) can pass into the guide tip lumen 19, through the collapsible guide sleeve 73, and out through the slit valve 75 and catheter tube opening 77.

Once this IAB is properly positioned, the guide wire (not shown) is withdrawn. Again, the guide sleeve can be dimensioned so that all of the guide wire, including any enlarged flexible tip structure, can pass therethrough.

When positive pressure is applied inside the balloon chamber 9, this causes at least the portion of the collapsible guide sleeve 73 in the vicinity of the region where the catheter tube 3 meets the distal end of the balloon to collapse. This part of the sleeve 73 would ordinarily hinder gas flow between the catheter tube lumen 5 and balloon chamber 9, since it is disposed in and reduces the cross-sectional area available for fluid flow through the tube lumen 5. However, when this part of the sleeve collapses, the effective area of the catheter tube lumen 5 is increased, and gas flow is improved. The slit valve 75 closes, preventing blood flow through the collapsible lumen 73. It is desirable to prevent blood from accumulating and coagulating in the proximal end of the collapsible lumen 73, since such clotting would prevent collapse of the lumen and would in fact obstruct the gas flow through lumen 5. However, coagulation at the distal end of the collapsible lumen might be acceptable, since it would not interfere with gas flow.

It is also possible to design the entire sleeve 73 to collapse, or to have the both the proximal and distal ends of the sleeve to collapse.

A stiffening member (not shown) is also provided to transfer the axial force applied by the physician along the catheter tube 3 to advance the IAB from the catheter tube 3 to the tip 17 of the balloon. This stiffening member can be located in the balloon chamber 9, embedded in the balloon membrane 7, or anywhere else, so long as sufficient force can be transferred to advance the balloon.

In the embodiment depicted in Fig. 16, the collapsible guide sleeve 73 runs within the balloon from the balloon's distal tip 17 into the catheter tube 3 to a position proximal to the location where the balloon membrane 7 is attached to the catheter wall 3. Since the guide sleeve 73 is open at both ends, the IAB can be slid over a guide wire, which passes through the tip lumen 19 in the tip 17 and exits through the catheter tube opening 77 and slit valve 75.

To provide an even better seal upon removal of the guide wire, the leaves of the slit valve 75, and/or portions of the interior of collapsible sleeve 73 can be coated with a thrombogenic material such as collagen. This IAB is easily advanced along the guide wire by passing the guide wire through the tip lumen 19 and slit valve 75, which opens to admit the wire, and then into the position for use. When it is time to remove the guide wire from the IAB, the coin purse valve 75 closes, and the slight amount of blood present in the collapsible guide sleeve 73 will, because of the thrombogenic material, undergo clotting that better seals the valve.

Instead of using a slit valve, one can provide thrombogenic material inside the guide sleeve so that when the wire is withdrawn, the sleeve lumen is sealed by clotting. Of course, this should be done in a manner which does not lead to clots floating in the bloodstream.

It is also possible to use the deflated balloon membrane itself to form a guide wire lumen. This is particularly advantageous because by eliminating the guide sleeve the size of the deflated balloon can be reduced.

As shown in Figs. 18A and 18B, the deflated balloon membrane 7 can be wrapped about the guide wire in various ways. In the pattern shown in Fig. 18A, the balloon membrane 7 is given a number of subfolds 79, which wrap around one another and enclose the guide wire 81 and a stiffening wire 83. In the pattern shown in Fig. 18B, the balloon member 7 is collapsed and flattened to provide a flap which is wrapped in a loose spiral around the guide wire 81 and stiffening wire 83. Of course, other folding configurations can be employed without departing from this aspect of the invention.

In an IAB catheter designed according to the embodiment of the invention shown in Figs. 18A or 18B, before insertion the balloon is folded so that the balloon membrane 7 surrounds the guide wire 81. These folds can be reliably maintained by annealing the balloon. The device is then inserted into the patient using known medical procedures. When the balloon is advanced through the vascular tree, the folded balloon membrane 7 insures that the IAB follows the guide wire. Once the balloon is properly positioned in the patient's aorta, the balloon can be inflated, and as it fills, it unfolds and no longer surrounds the guide wire 81 (it continues to surround the internal stiffening wire 83). The guide wire 81 now can be withdrawn from the patient.

To remove the IAB from the patient, the balloon chamber 9 is first evacuated, making the balloon as small as possible. The device is then withdrawn from the patient in a conventional manner.

As shown in Figs. 19 and 20, a device having the benefits of both dual- and single- lumen IAB's can be achieved by providing the IAB device 1 with a detachable guide sleeve 119 which can pass over a guide wire (not shown). Such a guide sleeve 119 is detachably affixed to the catheter tube 3 in a fashion such that during balloon insertion the guide sleeve helps the device follow the indwelling guide wire into the patient's aorta. Once positioned in the aorta, the guide sleeve 119 can be detached from the IAB and removed from the patient.

In this embodiment, the detachable guide sleeve 119 is joined by several low-shear bonds 117 to the region of the catheter tube 3 proximal to the proximal end of the balloon 13. Further, the guide sleeve 119 can, but need not extend up to the distal tip of the balloon 13. The balloon is then folded about the catheter tube 3 and either the guide sleeve 119, if that sleeve extends to the balloon, or the guide wire itself.

To use this embodiment of the invention, the guide wire is first positioned in the patient's aorta, and then the guide wire is threaded through the guide sleeve 119. Now, the entire assembly slides along the guide wire and follows the guide wire into the aorta. Once the IAB is properly positioned, the guide sleeve 119 can be detached and withdrawn from the patient. Withdrawal can be accomplished in a variety of ways. For example, if the guide sleeve 119 runs with the catheter tube 3 outside the body, tension can be applied to break these bonds by pulling on the length of guide sleeve extending from the patient.

Alternatively, the low shear bonds 117 can be broken by applying suitable force using the guide wire itself. By providing a guide wire having a distal end which is larger than the lumen of the guide sleeve 119, any force applied to the guide wire would be transferred to the guide sleeve 119, breaking the low shear bonds 117. Such an enlarged guide wire distal end could be tapered to facilitate passage through the arterial tree. A further benefit of this embodiment is that should a guide wire sleeve break, it would remain on the guide wire and not be lost in the body.

As previously explained in the description of related art, IAB's which do not have a guide-wire lumen inside the balloon require a stiffening wire or stylet. This features is necessary to position the balloon in the aorta. If this wire is made sufficiently small, i.e., 0.018", a balloon wrapped around such a stylet will be much smaller than a conventional design.

An IAB catheter designed in this manner has a catheter tube, balloon membrane, tip structure and stiffening wire. The balloon membrane can be attached to the catheter tube and tip structure in a conventional manner. To maintain a smooth catheter profile, which facilitates insertion, the stiffening wire can be affixed preferably to the inner wall, rather than the outer wall, of the catheter tube. This can be done by welding, bonding, or mechanically fastening the parts. Alternatively, the stiffening tube can actually be embedded either or both of the catheter tube and the tip structure.

It will be understood that the various guide structures which have been discussed can be used in catheters of many configurations, such as those having an eccentric balloon or multiple balloons.

Those of ordinary skill in the art will appreciate that numerous variations in materials and structure can be effected without departing from the spirit and scope of the claimed invention. Accordingly, this invention is not to be construed as limited to the embodiments set forth within the specification, but rather, extends to all devices falling within the following claims.

## Claims

1. An intra-aortic balloon catheter, comprising:
an inflatable balloon membrane (7) having a balloon chamber (9), a proximal end, and a distal end;
a catheter tube (3) having a catheter tube lumen (5); and
a guide sleeve (25) having a guide wire lumen (27), said guide sleeve (25) being affixed to an outside surface of said balloon membrane (7) without said guide sleeve (25) passing through said balloon membrane (7).

2. A retractable guiding tip for a catheter, comprising:
a shield collar (61) having cylindrical inner region, a proximal end, and a distal end;
a tubular guiding member (53) having a proximal end and a distal end (57), said tubular guiding member (53) being slidably received within said inner region so that said tubular guiding member can reciprocally move into and out of said shield collar (61); and
a stop means (55) for limiting movement of said tubular guiding member (53) into said shield collar (61).

3. An intra-aortic balloon catheter, comprising:
an inflatable balloon membrane (7) having a balloon chamber (9), a proximal end, and a distal end;
a catheter tube (3) having a catheter tube lumen (5), a proximal end, and a distal end, said tube lumen (5) being in fluid communication with said balloon chamber; and
a retractable guiding tip (51), said guiding tip (51) comprising a shield collar (61) having a clyindrical inner region, a proximal end, and a distal end (57), a tubular guiding member (53) having a proximal end and a distal end, said tubular guiding member (53) being slidably received within said inner region so that said tubular guiding member (53) can reciprocally move into and out of said shield collar (61); and
a stop means (55, 56) for limiting movement of said tubular guiding member (53) into said shield collar (61),
said retractable guiding tip (51) being disposed at said distal end of said balloon membrane, said guiding member (53) and said shield collar (61) being dimensioned so that regardless of how said guiding member (53) is positioned, said balloon chamber (9) is not in fluid communication with an environment outside said balloon chamber (9).

4. An intra-aortic balloon catheter, comprising:
a balloon membrane (7) having a balloon chamber (9), a proximal end, and a distal end;
a catheter tube (3) having a tube lumen (5), a proximal and a distal end, said proximal end of said balloon membrane (7) being attached to said catheter tube (3) in fluid-tight manner; and
a guide sleeve (25) having a guide wire lumen (27), said guide sleeve (25) being releasably attached to said catheter tube (3), wherein said guide sleeve (25) can be detached from said catheter tube (3) and removed.

5. An intra-aortic balloon, comprising:
a balloon membrane (7) having a balloon chamber (9), a proximal end, and a distal end;
a catheter tube (3) defined by a catheter tube wall and having a tube lumen (5), a proximal and a distal end, said proximal end of said balloon membrane (7) being attached to said catheter tube (3) in fluid-tight manner; and
a collapsible inner guide sleeve (73) having a proximal opening, a distal opening, and a guide wire lumen for accommodating a guide wire, said guide wire lumen running between said proximal opening and said distal opening, said distal opening being disposed at or near said distal end of said balloon membrane (7), said proximal opening of said inner tube being in said catheter tube lumen wall; and
sealing means (75) for sealing said proximal opening.

6. An intra-aortic balloon according to claim 5, wherein said sealing means comprises a slit valve (75).

7. A single-lumen, over-the-wire intra-aortic balloon, comprising:
a catheter tube (3) having a tube lumen (5), a proximal and a distal end;
a non-distensible balloon membrane (7) defining a collapsed balloon chamber (9);
balloon support means within said chamber (9), said balloon membrane (7) having a proximal end attached to said catheter tube (3) in fluid-tight manner, said balloon membrane (7) being wrapped, when said balloon chamber (9) is collapsed, so as to form an open passage approximately parallel to said balloon support means,
wherein a guide wire can be inserted through said open passage.

8. A method of guiding an intra-aortic balloon catheter, comprising the steps of:
providing an intra-aortic balloon catheter having a balloon membrane (7);
wrapping said balloon membrane (7) to form an open passage which will accommodate a guide wire;
causing said open passage to surround said guide wire; and
advancing said balloon catheter while said open passage surrounds said guide wire.
